# EUROPEAN PATENT SPECIFICATION

(11) **EP 3 052 649 B1**
(45) Date of publication and mention of the grant of the patent: **12.12.2018**
(21) Application number: 14786625.5
(22) Date of filing: 01.10.2014
(51) Int. Cl.: C12Q 1/6851, C12Q 1/6853

(54) **QUANTIFICATION OF MICRO RNA**
QUANTIFIZIERUNG VON MIKRO-RNA
QUANTIFICATION DE MICRO ARN

(30) Priority: 03.10.2013 EP 13004773
(43) Date of publication of application: 10.08.2016
(73) Proprietor: Biocartis NV, 2800 Mechelen (BE)
(72) Inventor: SERVOLI, Eva, CH-1005 Lausanne (CH); VAN DEN BOGAARD, Patrick, CH-1110 Morges (CH)
(74) Representative: De Clercq, Ann G. Y.
(86) International application number: PCT/EP2014/071076
(87) International publication number: WO 2015/049308

(56) References cited:
- WO-A2-03/060159
- BENES V ET AL: "Expression profiling of microRNA using real-time quantitative PCR, how to use it and what is available", METHODS : A COMPANION TO METHODS IN ENZYMOLOGY, ACADEMIC PRESS INC., NEW YORK, NY, US, vol. 50, no. 4, 28 January 2010 (2010-01-28), pages 244-249, XP026966677, ISSN: 1046-2023 [retrieved on 2010-01-28] cited in the application
- NICHOLAS REDSHAW ET AL: "A comparison of miRNA isolation and RT-qPCR technologies and their effects on quantification accuracy and repeatability", BIOTECHNIQUES, vol. 54, no. 3, March 2013 (2013-03), XP055160791, ISSN: 0736-6205, DOI: 10.2144/000114002
- E. MOKANY ET AL: "MNAzyme qPCR with Superior Multiplexing Capacity", CLINICAL CHEMISTRY, vol. 59, no. 2, February 2013 (2013-02), pages 419-426, XP055087024, ISSN: 0009-9147, DOI: 10.1373/clinchem.2012.192930
- "454 Sequencing System - Guidelines for Amplicon Experimental Design", Roche , July 2011 (2011-07), XP002716239, Retrieved from the Internet: URL:http://my454.com/downloads/my454/appli cations-info/454SequencingSystem_Guideline sforAmpliconExperimentalDesign_July2011.pd f
- BALLANTYNE ET AL: "Locked nucleic acids in PCR primers increase sensitivity and performance", GENOMICS, ACADEMIC PRESS, SAN DIEGO, US, vol. 91, no. 3, 14 February 2008 (2008-02-14), pages 301-305, XP022482717, ISSN: 0888-7543, DOI: 10.1016/J.YGENO.2007.10.016
- J Brownie ET AL: "The elimination of primer-dimer accumulation in PCR", Nucleic Acids Research, vol. 25, no. 16, 15 August 1997 (1997-08-15), pages 3235-3241, XP055296897, DOI: 10.1093/nar/25.16.3235

## Description

The present invention relates to a method for quantifying a plurality of microRNAs (miRNAs).

MicroRNAs (miRNAs) are small, non-coding RNAs that regulate various biological processes, primarily through interaction with messenger RNAs (mRNAs). The levels of miRNAs in blood or serum have been associated with various pathological conditions including cardiovascular, autoimmune and neurodegenerative diseases or cancers. In this respect, miRNAs are now considered as promising diagnostic and prognostic biomarkers for various pathophysiological conditions.

The use of miRNAs as biomarkers requires standardization of sample collection and miRNAs extraction protocols. The extracted miRNAs are commonly transcripted into their corresponding cDNAs by reverse transcription. One of the issues of using miRNAs as biomarkers is that the measured concentration of cDNAs has to be correlated to the initial amount of miRNAs in the sample to provide both accurate and biologically relevant results.

To that end, a mainstream method to determine the miRNAs content of a sample is to repeat individual detection reaction of each cDNA, each reaction being performed in an individual tube comprising a specific pair of primers targeting one of the cDNAs of interest. Thus, each cDNA is analyzed individually to deduce information regarding the presence of the corresponding miRNA in the sample analyzed.

However in such a method involving individual reactions as mentioned above, the screening of a panel of miRNAs requires considerable hands-on time and large volumes of reagents, which makes the assay expensive and not suitable for routinely clinical tests.

Therefore, there is a need for a method allowing the analysis of a sample comprising multiple miRNAs to provide an overview of the amounts of the miRNAs comprised of the sample in a more convenient way.

Benes et al. (Methods: a comparison to methods in enzymology, Acad. Press Inc. New York, NY, US, (2010) 50(4):244-249) describes expression profiling of microRNA using real-time quantitative PCR. The method of Benes et al. comprises reverse transcription using a tagged miRNA specific primer, followed by amplification using another miRNA specific primer together with a universal primer annealing to the tag sequence. This method is not compatible with universal reverse transcription.

Mokany et al. (Clinical Chemistry (2013) 59(2):419-426) discloses that MNAzymes can be linked to PCR to provide a highly specific method for target detection and quantification.

Redshaw et al. (Biotechniques (2013) 54(3):155-164) compares miRNA isolation and RT-qPCR technologies and their efforts on quantification accuracy and repeatability. Redshaw et al. discloses a method involving poly(A) tailing of mature miRNA followed by reverse transcription using a polyT primer with a universal tag and then SYBR Green qPCR using miRNA-specific forward and reverse primers containing LNAs. This method does not involve a second PCR amplification, let alone at a higher annealing temperature than the first PCR amplication.

Brownie et al. (Nucl. Acids Res. (1997) 25(16):3235-3241) elaborates on the elimination of primer-dimer accumulation in PCR but is silent on miRNA quantification and RNA amplification.

The present invention aims to remedy all or part of the disadvantages mentioned above.

The present invention fulfills these objectives by providing a method for quantifying a plurality of miRNAs, said method comprising the successive step of:
a. Performing a universal reverse transcription protocol to provide a sample comprising a plurality of cDNAs, each cDNA corresponding to a miRNA;
b. Contacting the sample with a plurality of pairs of tagged primers and a pair of universal primers, each tagged primer comprising a specific moiety and a tagged moiety, one of the specific moiety being designed for hybridizing to a cDNA and each tagged moiety being designed for hybridizing to one of the universal primers;
c. Amplifying each cDNA by performing a first PCR amplification using a pair of tagged primers, wherein annealing step of the first PCR amplification is performed at a first annealing temperature T1, in order to provide a plurality of DNAs comprising the tagged moieties;
d. Amplifying each DNA by performing a second PCR amplification using the pair of universal primers, wherein annealing step of the second PCR amplification is performed at a second annealing temperature T2, said second annealing temperature T2 being superior to the first annealing temperature T1;
e. Determining the concentration of each cDNA in the sample to deduce the concentration of each miRNA.

Thus, the present invention solves the problem mentioned above by providing a method for quantifying simultaneously in one pot a plurality of cDNA comprised in a sample, each cDNA corresponding to a miRNA in order to deduce the absolute concentration of each miRNA. The sample is contacted with a plurality of pairs of tagged primers, each pairs of tagged primers being designed to amplify only one of the cDNAs comprised in the sample. Thus, a plurality of cDNAs is amplified simultaneously in one assay, whereas this feature was not possible according to the known methods using multiple individual assay detection when it comes to analyze a plurality of miRNAs. In this respect, the method according to the present invention provides information about the concentration of the plurality of miRNAs in the sample analyzed with a minimum of hands-on time compared to the method described in the prior art previously mentioned. Advantageously, each tagged primer further comprises a tagged moiety so as to transfer one of the tagged moieties from one tagged primer to each strand of DNA synthetized during a first PCR amplification. During a second PCR amplification, a pair of universal primers is capable of hybridizing to the tagged moieties comprised in each DNA in order to amplify each DNA with the same efficiency. This feature allows maintaining the respective concentrations of the plurality of DNAs amplified during the first PCR amplification thereby providing a correlation between the concentration of the plurality DNA and the concentration of the corresponding cDNA initially present in the analyzed sample, so that the concentration of the miRNAs can be deduced therefrom. Thus, the present invention provides absolute quantification of the plurality of cDNAs to determine their absolute concentrations. Additionally, the method allows the determination of relative ratio between the cDNAs thereby giving access to the relative ratio of miRNAs.

As indicated above, step a) of the method of the invention further comprises a universal reverse transcription protocol in order to provide a plurality of cDNA. The miRNAs are reverse transcribed using a universal reverse transcription method, wherein the miRNAs are first tailed with a common sequence and then reverse transcribed using a universal primer targeting said tail. For example, adenosine nucleotides may be added to the 3' end of the miRNAs upon adding a poly(A) polymerase, such as *E. coli* Poly(A) Polymerase, and then, a primer comprising an oligo(dT) sequence is used for the reverse transcription. Another exemplary universal reverse transcription method is the miQPCR method as described in Benes and Castoldi (2010. Methods 50:244-249; 3.2 cDNA synthesis by tailing RNAs, 2^{nd} paragraph), which exploits the activity of T4 RNA ligase 1 to covalently attach the 3'-hydroxyl group of miRNAs to the 5'-phosphate group of a RNA/DNA linker adaptor, which comprises a universal primer-binding sequence. The extended miRNAs are then reverse transcribed by using a universal primer complementary to the 3'-end of the linker.

Universal reverse transcription is particularly useful if several different miRNAs need to be analyzed from a small amount of starting material.

In an embodiment, the concentration of each cDNA is provided by using encoded particles, said encoded particles being functionalized with at least a partzyme capable of forming MNAzyme complex. Advantageously, each particle is encoded with a unique code corresponding to the DNA to be detected thereon, thereby allowing detection of a plurality of DNAs simultaneously to provide the concentration of a plurality of cDNA.

According to an embodiment, a PCR dye is added to monitor the first PCR amplification and/or the second PCR amplification. Advantageously, the PCR dye is designed for emitting a fluorescent signal when contacting double stranded DNA, the fluorescent signal being proportional to the amount of double stranded DNA. Thus, the PCR dye allows monitoring the amplification of the double stranded DNA.

In an embodiment, a control nucleic acid is added to the sample in order to control the amplification rate during the first PCR amplification and/or the second PCR amplification. The control nucleic acid allows preventing the saturation of the DNA corresponding to the plurality of cDNA that may occurs during the first PCR amplification and/or the second PCR amplification. In this respect, the control nucleic acid maintains the correlation between the concentration of the plurality DNA and the concentration of the corresponding cDNA initially present in the sample.

According to an embodiment, the first annealing temperature T1 is comprised between about 56°C and about 61°C, preferentially between about 58°C and about 61°C.

In an embodiment, the second annealing temperature T2 is comprised between about 65°C and about 70°C, preferentially between about 66°C and about 68°C.

According to an embodiment, the tagged primer comprises LNA. Advantageously, Locked Nucleic Acids (LNA) ensure high specificity during the hybridization of one of the tagged primers to the cDNA and therefore guarantee that the first amplification reaction occurs only for the cDNA of interest.

The present invention is further illustrated by the following detailed description which represents an exemplary and explanatory embodiment of a method for quantifying a plurality of miRNA.

In the present embodiment, the method according to the present invention aims at quantifying the concentration of two miRNA, miRNA1 and miRNA2, both miRNAs being isolated from a serum. Those miRNAs, miRNA1 and miRNA2, are two recognized biomarkers, particularly relevant for cancers diagnostic.

The method is initiated by providing two cDNA, cDNA1 and cDNA2 corresponding respectively to the reverse transcript of miRNA1 and miRNA2. To that end, miRNA1 and miRNA2 are contacted with a standard master mix containing optimized concentration of the required reagents such as MgCl₂, dNTPs (dATP, dCTP, dGTP, dTTP), recombinant RNase inhibitor protein, qScript reverse transcriptase, random primers, oligo(dT) primer and stabilizers. Then, a standard protocol is a two-step protocol, comprising of polyadenilation (one hour at 37°C, followed by inactivation) and universal reverse transcription (20 minutes at 42°C, followed by inactivation) to provide a cDNA solution containing cDNA1 and cDNA2.

Subsequently, cDNA1 and cDNA2 resulting from the reverse transcription reactions are amplified via a first PCR amplification to provide a plurality of DNA1 and DNA2 issued respectively from cDNA1 and cDNA2. Then, a second PCR amplification is capable of amplifying the DNA1 and the DNA2. The first PCR amplification and the second PCR amplification are performed successively in the same PCR chamber. Advantageously, the first PCR amplification and the second PCR amplification are simultaneously completed respectively from the plurality of cDNA and from the plurality of DNA comprised in the PCR chamber.

To perform the first PCR amplification and the second PCR amplification, cDNA1 and cDNA2 are simultaneously contacted with a pair of universal primers and with two pairs of tagged primers, a pair of tagged primers 1 and a pair of tagged primers 2.

In the method according to the present embodiment, each the tagged primer is designed for annealing to the cDNA or to a DNA strand complementary to the cDNA, at a first annealing temperature T1 to allow the first PCR amplification. After the first PCR amplification, each universal primer is designed for annealing to one of the DNA strand at a second annealing temperature T2 to permit the second PCR amplification.

One of the tagged primers 1 and one of the tagged primers 2 are designed for being specifically hybridized respectively to cDNA1 and cDNA2.

In each pair of tagged primer 1, one of the tagged primers 1 comprises a tagged moiety A and a specific moiety 1, the other tagged primer 1 comprising a tagged moiety B and a specific moiety 2. Concerning the tagged primers 2, in each pair of tagged primer 2, one of the tagged primers 2 comprises the tagged moiety A and a specific moiety 2, the other tagged primer 2 comprising the tagged moiety B and a specific moiety 2.

Thus, both tagged primers 1 and 2 comprise the tagged moiety A and the tagged moiety B, said tagged moieties A and B being designed for being hybridized to one of the universal primers. In the present case, the pair of universal primers comprises a universal primer A and a universal primer B designed for respectively hybridizing to the tagged moiety A and to the tagged moiety B.

Then, a reaction mixture is prepared to perform the first PCR amplification and the second PCR amplification. To that end, 4 ul of cDNA solution comprising cDNA1 and cDNA2 is combined in the PCR chamber with 0.4 ul of tagged primers 1, 0.4 ul of tagged primers 2, 200 nM of universal primer A, 200 nM of universal primer B, 7 ul of Sso Fast EvaGreen® qPCR Master Mix as a PCR dye (commercialized by BIORAD) and PCR grade water up to 10 ul. Additionally, a control nucleic acid is added to the reaction mixture at a concentration ten times higher than cDNA1 and cDNA2, in order to control the amplification rate during the first PCR amplification and/or the second PCR amplification. In the present case, the control nucleic acid is a cDNA corresponding to a Cel-miR-39. Subsequently, the cDNA1 and the cDNA2 comprised in the reaction mixture contained in the PCR chamber are amplified during the first PCR amplification. The first PCR amplification is initiated by 5 minutes at 95°C, followed by 15 minutes at the first annealing temperature T1 that is 60°C in the present embodiment. Then, the first PCR amplification comprises eight repeated cycles, each cycle including 10 seconds at 95°C followed by 30 seconds at 60°C. During these cycles, the concentration of DNA 1 and DNA2 is monitored by measuring the fluorescence signal emitted by the PCR dye consisting of the EvaGreen® dye in the present case.

Then, DNA1 and DNA2 provided via the first PCR amplification are amplified during the second PCR amplification. To that end, the second PCR amplification comprises forty repeated cycles, each cycle including 10 minutes at 95°C followed by 30 seconds at the second annealing temperature T2 that is 67°C in the present case, and finally 30 seconds at 72°C.

Advantageously, in the present embodiment the method further comprises a quantification detection step to provide the concentration of each cDNA so as to deduce the concentration of each miRNA in the sample. To that end, encoded particle designed for performing MNAzyme detection are used wherein each encoded particles is designed for detecting a specific DNA. The kinetic of the detection provide quantitative information about the concentration of cDNA initially present in the reaction mixture. Thus, the determination of the concentration of cDNA1 and cDNA2 in the reaction mixture allow deducing the concentration of miRNA1 and miRNA2 present in the analyzed serum.

Other embodiments of the invention will be apparent to those skilled in the art from consideration of the specification and practice of the invention disclosed herein. It is intended that the specification and examples be considered as exemplary only, with the true scope of the invention being indicated by the following claims.

## Claims

1. A method for quantifying a plurality of miRNAs, said method comprising the successive step of:
a. Performing a universal reverse transcription protocol to provide a sample comprising a plurality of cDNAs, each cDNA corresponding to a miRNA;
b. Contacting the sample with a plurality of pairs of tagged primers and a pair of universal primers, each tagged primer comprising a specific moiety and a tagged moiety, one of the specific moiety being designed for hybridizing to a cDNA and each tagged moiety being designed for hybridizing to one of the universal primers;
c. Amplifying each cDNA by performing a first PCR amplification using a pair of tagged primers, wherein annealing step of the first PCR amplification is performed at a first annealing temperature T1, in order to provide a plurality of DNAs comprising the tagged moieties;
d. Amplifying each DNA by performing a second PCR amplification using the pair of universal primers, wherein annealing step of the second PCR amplification is performed at a second annealing temperature T2, said second annealing temperature T2 being superior to the first annealing temperature T1;
e. Determining the concentration of each cDNA in the sample to deduce the concentration of each miRNA.

2. A method according to claim 1, wherein the concentration of each cDNA is provided by using encoded particles, said encoded particles being functionalized with at least a partzyme capable of forming MNAzyme complex.

3. A method according to any one of claims 1 or 2, wherein a PCR dye is added to monitor the first PCR amplification and/or the second PCR amplification.

4. A method according to any one of claims 1 to 3, wherein a control nucleic acid is added to the sample in order to control the amplification rate during the first PCR amplification and/or the second PCR amplification.

5. A method according to any one of claims 1 to 4, wherein the first annealing temperature T1 is comprised between about 56°C and about 61°C, preferentially between about 58°C and about 61°C.

6. A method according to any one of claims 1 to 5, wherein the second annealing temperature T2 is comprised between about 65°C and about 70°C, preferentially between about 66°C and about 68°C.

7. A method according to any one of claims 1 to 6, wherein the tagged primer comprises LNA.

## Patentansprüche

1. Verfahren zum Quantifizieren einer Vielzahl von miRNAs, wobei das Verfahren aufeinanderfolgend die nachstehenden Schritte umfasst:
a. Durchführen eines universellen reversen Transkriptions-Protokolls, um eine Probe bereitzustellen, umfassend eine Vielzahl von cDNAs, wobei jede cDNA einer miRNA entspricht;
b. Inkontaktbringen der Probe mit einer Vielzahl von Paaren getaggter Primer und einem Paar universeller Primer, wobei jeder getaggte Primer eine spezifische Einheit und eine getaggte Einheit umfasst, wobei eine der spezifischen Einheiten zum Hybridisieren an eine cDNA entworfen ist und jede getaggte Einheit zum Hybridisieren an einen der universellen Primer entworfen ist;
c. Amplifizieren jeder cDNA mittels Durchführung einer ersten PCR-Amplifikation unter Verwendung eines Paares von getaggten Primern, wobei der Schritt des Annealens der ersten PCR-Amplifikation bei einer ersten Annealingtemperatur T1 durchgeführt wird, um eine Vielzahl von DNAs bereitzustellen, die die getaggten Einheiten umfassen;
d. Amplifizieren jeder DNA mittels Durchführung einer zweiten PCR-Amplifikation unter Verwendung des Paares von universellen Primern, wobei der Schritt des Annealens der zweiten PCR-Amplifikation bei einer zweiten Annealingtemperatur T2 durchgeführt wird, wobei die zweite Annealingtemperatur T2 höher als die erste Annealingtemperatur T1 ist;
e. Bestimmen der Konzentration jeder cDNA in der Probe, um die Konzentration jeder miRNA abzuleiten.

2. Verfahren nach Anspruch 1, wobei die Konzentration jeder cDNA bereitgestellt wird, indem codierte Partikel verwendet werden, wobei die codierten Partikel mit mindestens einem Partzym funktionalisiert sind, das imstande ist, einen MNAzym-Komplex zu bilden.

3. Verfahren nach einem der Ansprüche 1 oder 2, wobei ein PCR-Farbstoff hinzugefügt wird, um die erste PCR-Amplifikation und/oder die zweite PCR-Amplifikation zu überwachen.

4. Verfahren nach einem beliebigen der Ansprüche 1 bis 3, wobei zu der Probe eine Kontrollnukleinsäure hinzugefügt wird, um die Amplifikationsrate während der ersten PCR-Amplifikation und/oder der zweiten PCR-Amplifikation zu kontrollieren.

5. Verfahren nach einem der Ansprüche 1 bis 4, wobei die erste Annealingtemperatur T1 zwischen etwa 56°C und etwa 61°C, präferentiell zwischen etwa 58°C und etwa 61°C liegt.

6. Verfahren nach einem der Ansprüche 1 bis 5, wobei die zweite Annealingtemperatur T2 zwischen etwa 65°C und etwa 70°C, vorzugsweise zwischen etwa 66°C und etwa 68°C liegt.

7. Verfahren nach einem der Ansprüche 1 bis 6, wobei der getaggte Primer LNA umfasst.

## Revendications

1. Procédé de quantification d'une pluralité de miARN, ledit procédé comprenant les étapes successives de :
a. conduite d'un protocole de transcription inverse universel pour fournir un échantillon comprenant une pluralité d'ADNc, chaque ADNc correspondant à un miARN ;
b. mise en contact de l'échantillon avec une pluralité de paires d'amorces étiquetées et une paire d'amorces universelles, chaque amorce étiquetée comprenant un fragment spécifique et un fragment étiqueté, l'un du fragment spécifique étant conçu pour s'hybrider à un ADNc et chaque fragment étiqueté étant conçu pour s'hybrider à l'une des amorces universelles ;
c. amplification de chaque ADNc par conduite d'une première amplification par PCR au moyen d'une paire d'amorces étiquetées, l'étape d'hybridation de la première amplification par PCR étant conduite à une première température d'hybridation T1, afin de fournir une pluralité d'ADN comprenant les fragments étiquetés ;
d. amplification de chaque ADN par conduite d'une deuxième amplification par PCR en utilisant la paire d'amorces universelles, l'étape d'hybridation de la deuxième amplification par PCR étant conduite à une deuxième température d'hybridation T2, ladite deuxième température d'hybridation T2 étant supérieure à la première température d'hybridation T1 ;
e. détermination de la concentration de chaque ADNc dans l'échantillon pour déduire la concentration de chaque miARN.

2. Procédé selon la revendication 1, dans lequel la concentration de chaque ADNc est fournie en utilisant des particules codées, lesdites particules codées étant fonctionnalisées avec au moins un partzyme capable de former un complexe MNAzyme.

3. Procédé selon l'une quelconque des revendications 1 ou 2, dans lequel un colorant de PCR est ajouté pour surveiller la première amplification par PCR et/ou la deuxième amplification par PCR.

4. Procédé selon l'une quelconque des revendications 1 à 3, dans lequel un acide nucléique témoin est ajouté à l'échantillon afin de contrôler le taux d'amplification pendant la première amplification par PCR et/ou la deuxième amplification par PCR.

5. Procédé selon l'une quelconque des revendications 1 à 4, dans lequel la première température d'hybridation T1 est comprise entre environ 56 °C et environ 61 °C, de préférence entre environ 58 °C et environ 61 °C.

6. Procédé selon l'une quelconque des revendications 1 à 5, dans lequel la deuxième température d'hybridation T2 est comprise entre environ 65 °C et environ 70 °C, de préférence entre environ 66 °C et environ 68 °C.

7. Procédé selon l'une quelconque des revendications 1 à 6, dans lequel l'amorce étiquetée comprend un acide nucléique bloqué (LNA).
